# EUROPÄISCHE PATENTSCHRIFT

(11) **EP 1 862 936 B1**
(45) Veröffentlichungstag und Bekanntmachung des Hinweises auf die Patenterteilung: **26.02.2020**
(21) Anmeldenummer: 07104308.7
(22) Anmeldetag: 16.03.2007
(51) Int. Cl.: G06F 21/42, G06F 21/46, G06F 21/62, G06F 21/60, G06F 21/34, G16H 10/60

(54) **VERFAHREN ZUR IDENTIFIKATION EINES PATIENTEN ZUM SPÄTEREN ZUGRIFF AUF EINE ELEKTRONISCHE PATIENTENAKTE DES PATIENTEN MITTELS EINER KOMMUNIKATIONSEINRICHTUNG EINER ANFRAGENDEN PERSON**
METHOD FOR IDENTIFYING A PATIENT WITH LATER ACCESS TO ELECTRONIC RECORDS OF THE PATIENT VIA A COMMUNICATION DEVICE FOR A REQUESTING AGENT
PROCÉDÉ D'IDENTIFICATION D'UN PATIENT DESTINÉ À LA SAISIE ULTÉRIEURE SUR UN DOSSIER PATIENT ÉLECTRONIQUE DU PATIENT GRÂCE À UN DISPOSITIF DE COMMUNICATION D'UNE PERSONNE LE DEMANDANT

(30) Priorität: 31.05.2006 DE 102006025763
(43) Veröffentlichungstag der Anmeldung: 05.12.2007
(73) Patentinhaber: Siemens Healthcare GmbH, 91052 Erlangen (DE)
(72) Erfinder: Haider, Sultan, Erlangen 91052 (DE); Heidenreich, Georg, Erlangen 91056 (DE)

(56) Entgegenhaltungen:
- EP-A- 1 646 254
- WO-A-2004/052026
- WO-A-2004/104898
- WO-A1-03/093956
- US-A1- 2002 029 157
- US-A1- 2003 074 564

## Beschreibung

Die Erfindung betrifft ein Verfahren zur Identifikation eines Patienten zum späteren Zugriff auf eine elektronische Patientenakte des Patienten mittels einer Kommunikationseinrichtung einer anfragenden Person, welche Patientenakte in einer Datenbank über einen der Identifikation des Patienten dienenden Primärschlüssel, dem eindeutig wenigstens ein Sekundärschlüssel zugeordnet ist, gespeichert ist.

Zu einer umfassenden, qualitativ hochwertigen Betreuung eines Patienten in einem Gesundheitssystem ist es vorteilhaft, wenn die Daten des Patienten in einer idealerweise von der ganzen Welt aus erreichbaren elektronischen Patientenakte gespeichert sind. Solche Patientenakten enthalten dann beispielsweise neben der Krankengeschichte des entsprechenden Patienten Bildaufnahmen oder Testergebnisse.

Die einzelnen elektronischen Patientenakten entsprechen dabei Einträgen in einer Datenbank, die intern über einen Primärschlüssel die einzelnen Patientenakten individualisiert. Jeder Patientenakte ist folglich ein Primärschlüssel eindeutig zugeordnet. Die Primärschlüssel werden jedoch automatisch vergeben und stehen in keinem konkreten Zusammenhang zum Patienten selber. Der Primärschlüssel ermöglicht also das Auffinden einer Patientenakte eines speziellen Patienten, kennzeichnet jedoch den Patienten selber nicht. Daher wurde vorgeschlagen, Sekundärschlüssel zu verwenden, die in konkretem Zusammenhang mit der Person des Patienten stehen. Die Verwendung von Namen und Geburtsdaten erweist sich jedoch aufgrund der Nichteindeutigkeit allgemein als nicht ideal geeignet.

Es wurde daher vorgeschlagen, beispielsweise eine Krankenversicherungskarte als Träger des Primärschlüssels oder eines Sekundärschlüssels zu verwenden. Zum Auslesen einer solchen Krankenversicherungskarte sind zusätzliche Geräte erforderlich, die auch beispielsweise ein Notarzt ständig mit sich führen müsste, um einen Patienten zweifelsfrei zu identifizieren, das bedeutet, den Primärschlüssel der elektronischen Patientenakte des Patienten zu erfahren. Insbesondere in Notfällen kommt es dabei auf eine schnelle und sichere Identifizierung des Patienten an, da die Verwendung falscher Gesundheitsdaten durch einen Heilberufler leicht zu Komplikationen führen kann.

In WO 2004/052 026 A2 wird die Authentisierung eines Benutzers gegenüber einem Server mit einer Dateistruktur beschrieben. Der Benutzer übermittelt dabei über ein Mobilgerät eine Identifikationsinformation, welche das Mobilgerät identifiziert und es von anderen Mobilgeräten unterscheidet. Auf Grundlage dieser Information kann der Benutzer Zugriff auf die Dateistruktur erhalten, so dass ein Zugriff auch ohne die korrekte User-ID und/oder das korrekte Passwort möglich ist.

US 2002/0 029 157 A1 beschreibt eine computergestützte Datenbank für medizinische und biografische Daten sowie für diagnostische Informationen. Die auf der Datenbank enthaltenen Informationen können von weiteren Rechnern über einen Fernzugriff eingefügt oder ausgelesen werden.

In EP 1 646 254 A1 wird eine Methode zur Identifizierung und/oder zur Authentifizierung eines Benutzers eines Mobilgeräts in einem Server anhand von Fingerabdrücken des Benutzers beschrieben. Die Fingerabdrücke werden dabei durch haptische Eingabemittel des Mobilgeräts mit einem Fingerabdruckleser im Mobilgerät gelesen. Dazu wird der Benutzer aufgefordert, Auswahlen in einem Menü des Servers mit den haptischen Eingabemitteln durchzuführen, wobei ein gleichzeitiges Lesen der Fingerabdrücke des Benutzers durch die haptischen Eingabemittel erfolgt. Zur Identifikation oder Authentifizierung des Benutzers erfolgt ein Vergleich der eingelesenen Fingerabdrücke mit Referenzfingerabdrücken.

US 2003/0 074 564 A1 betrifft ein Verfahren zur Verwaltung und zum Zugriff auf elektronische medizinische Akten, bei dem Sicherheits- und Anonymisierungsaspekte berücksichtigt werden. Dazu wird ein Internetportal vorgeschlagen, in dem Akten doppelt verschlüsselt in einer Datenbank gespeichert sind. Jeder Person wird dabei ein Identifikator zugewiesen, welcher beispielsweise auf einer Karte abgedruckt sein kann. Weiterhin hat jede Person einen zweiten, einzigartigen Identifikator, welchen sie verwenden kann, wenn die Karte mit dem ersten Identifikator nicht verfügbar ist. Beide Identifikatoren ermöglichen dabei Zugriff auf die in der Datenbank des Servers gespeicherten Daten. Nach Senden des als globaler Schlüssel dienenden ersten Identifikators oder des einen einzigartigen persönlichen Verschlüsselungsschlüssel darstellenden zweiten Identifikators an den Server übersendet dieser die gespeicherten Daten an die anfragende Person.

In WO 03/093 956 A1 wird ein Verfahren zur Speicherung von sensiblen Informationen beschrieben, bei dem die Informationen leicht zu erhalten, aber nicht einem Individuum zuordenbar sein sollen. Dazu werden ein interner Identifikator sowie zwei separate Datenbanken verwendet. In einer der Datenbanken werden die Informationen zusammen mit einem zweiten Identifikator gespeichert. In der anderen Datenbank ist der zweite Identifikator mit einem einer Person zugeordneten persönlichen Identifikator gespeichert, wobei zwischen dem zweiten Identifikator und dem persönlichen Identifikator keine Zuordenbarkeit außerhalb der Datenbank besteht. Über den zweiten Identifikator erfolgt eine Zuordnung der sensiblen Informationen zu dem persönlichen Identifikator. Bei Übersenden eines persönlichen Identifikators erfolgt eine Verknüpfung des persönlichen Identifikators mit den hinterlegten Daten, wenn die Anfrage von einer autorisierten Person übermittelt wurde.

Der Erfindung liegt daher die Aufgabe zugrunde, ein Verfahren zur Identifikation eines Patienten, also zum Auffinden des Primärschlüssels eines Patienten, anzugeben, welches es ohne komplexe, zusätzliche Hardware ermöglicht, möglichst zweifelsfrei die Identität eines Patienten festzustellen.

Diese Aufgabe wird erfindungsgemäß durch ein Verfahren gemäß Anspruch 1 gelöst.

Eine solche einen Teilnehmer eines drahtlosen Kommunikationsnetzes kennzeichnende Teilnehmerinformation, die als Sekundärschlüssel verwendet werden kann, kann beispielsweise eine Nummer einer SIM-Karte und/oder eine Rufnummer des Patienten und/oder eine Gerätenummer eines zur Kommunikation in dem drahtlosen Kommunikationsnetz dienenden Mobilterminals des Patienten sein. Ein Mobilterminal im Sinne der Erfindung ist beispielsweise ein Mobiltelefon, aber auch jedes andere mobile Gerät, das zur drahtlosen Kommunikation in dem drahtlosen Kommunikationsnetz geeignet ist, beispielsweise netzfähige PDAs.

Erfindungsgemäß wird also vorteilhafterweise mit dem Mobilterminal eine Gerätschaft verwendet, die ein Patient ohnehin jederzeit bei sich führt. Zur Nutzung des drahtlosen Kommunikationsnetzes sind das Mobilterminal und eine darin einsteckbare SIM-Karte erforderlich. Jedes Mobilterminal besitzt dabei eine eigene, eindeutige, es von allen anderen Mobilterminals unterscheidende Gerätenummer, beispielsweise die sogenannte IMEI-Nummer (International Mobile Equipment Identity). Die IMEI ist eine eindeutige, 15-stellige Seriennummer, anhand derer jedes GSM- oder UMTS-Endgerät eindeutig identifiziert werden kann. Auch eine SIM-Karte weist eine eindeutige SIM-Kartennummer auf, und durch sie erhält das Mobilterminal die - ebenso eindeutige - Rufnummer des Patienten. Damit sind schon drei Beispiele für Teilnehmerinformationen genannt, welche für jeden Teilnehmer an dem drahtlosen Kommunikationsnetzwerk kennzeichnend sind und die dieser Teilnehmer zwangsläufig mit sich führt. Die der Erfindung zugrunde liegende Idee ist es nun, diese ohnehin vorhandene, den Patienten eindeutig identifizierende Teilnehmerinformation als einen Sekundärschlüssel zu verwenden, der dem Primärschlüssel eindeutig zugeordnet wird. Dabei können auch mehrere, insbesondere alle genannten, Teilnehmerinformationen als Sekundärschlüssel eindeutig dem Primärschlüssel zugeordnet sein.

Vorteilhafterweise kann dann zudem die entsprechende zugehörige Hardware, also das Mobilterminal, genutzt werden, um die tatsächliche Identität, also den Primärschlüssel, des Patienten festzustellen. Dazu findet eine Kommunikation mit einem Portal statt, in dem die Zuordnung der jeweiligen Sekundärschlüssel zu den Primärschlüsseln abgelegt ist. Dabei kann es sich zum einen um die Einrichtung handeln, in der auch die Datenbank mit den elektronischen Patientenakten abgelegt ist, wobei auch die Sekundärschlüssel in dieser Datenbank gespeichert sind. Es ist jedoch auch denkbar, dass eine auf dem Portal abgelegte zweite Datenbank verwendet wird, worin der oder die Sekundärschlüssel dem Primärschlüssel zugeordnet sind. Dann kann die die elektronischen Patientenakten enthaltende Datenbank auf einer anderen Einrichtung abgelegt sein. Etwas Derartiges erweist sich insbesondere als vorteilhaft, wenn eine Zusammenarbeit mit einem Betreiber eines solchen drahtlosen Kommunikationsnetzes gesucht wird, jener allerdings, beispielsweise aus Datenschutzgründen, keinerlei Zugriff auf die elektronischen Patientenakten selber erhalten soll. Dann liegen folglich zwei Datenbanken vor, wobei in der auf dem Portal abgelegten zweiten Datenbank problemlos die Zuordnung eines Sekundärschlüssels zu einem Primärschlüssel erfolgen kann.

Die Kommunikationseinrichtung der anfragenden Person kann jede Art von Kommunikationseinrichtung sein, die zum Zugriff auf die elektronische Patientenakte ausgebildet ist. Es kann eine spezielle Kommunikationseinrichtung zum Zugriff auf die Patientenakte vorgesehen sein, es ist jedoch auch möglich, bekannte Kommunikationseinrichtungen wie Workstation-Computer, Laptops, Handhelds oder sogar Mobiltelefone so auszugestalten, beispielsweise über ein Softwaremittel, dass der Zugriff auf elektronische Patientenakten in der Datenbank ermöglicht wird.

Zur Identifikation des Patienten, also zum Auffinden des Primärschlüssels, der die Akte des Patienten auffindbar macht, ist erfindungsgemäß eine Kommunikation mit dem Portal und einem Mobilterminal vorgesehen. Dabei wird der Sekundärschlüssel übertragen, welcher problemlos einem Primärschlüssel zuordenbar ist bzw. umgekehrt. Der Primärschlüssel kann dann beispielsweise an die Kommunikationseinrichtung einer anfragenden Person übertragen werden. Eine solche anfragende Person ist erfindungsgemäß ein Heilberufler, beispielsweise ein Arzt, Rettungssanitäter, usw. Zur konkreten Ausgestaltung der Übertragung und der daraus folgenden Identifikation eines Patienten sind erfindungsgemäß verschiedene Ausgestaltungen denkbar.

So kann vorgesehen sein, dass zur Identifikation des Patienten ein Mobilterminal des Patienten oder ein mit einer SIM-Karte des Patienten versehenes Mobilterminal verwendet wird, welches Daten an das Portal sendet und/oder von dem Portal empfängt. Die Identifikation erfolgt hierbei mittels Daten, die von dem Mobilterminal an das Portal oder von dem Portal an das Mobilterminal gesendet werden. Dabei ist es wichtig, dass entweder das Mobilterminal des Patienten (Mobilterminalgerätenummer) oder die SIM-Karte des Patienten (SIM-Kartennummer, Rufnummer) oder beide Verwendung finden, so dass wenigstens einer der Sekundärschlüssel übertragen werden kann bzw. überprüft werden kann.

In weiterer Ausgestaltung der Erfindung ist vorgesehen, dass von dem Mobilterminal den oder die Sekundärschlüssel umfassende Daten, insbesondere in Form einer SMS oder mittels GPRS, an das Portal übersendet werden, aufgrund des oder der Sekundärschlüssel durch das Portal der Primärschlüssel ermittelt und unter Nutzung weiterer in den Daten umfasster Informationen der Kommunikationseinrichtung übermittelt wird. Hierunter fallen beispielsweise die Fälle der Vorwärtsidentifikation. Bei einer Vorwärtsidentifikation initiiert im weiteren Sinne der Patient selber die Authentifizierung, in diesem Falle, indem beispielsweise eine SMS oder ein GPRS-Datenpaket an das Portal übersendet wird. Die Daten umfassen dabei den oder die Sekundärschlüssel, wobei beispielsweise bei einer SMS die Rufnummer selber automatisiert in den Daten enthalten ist. Zusätzlich zu den Sekundärschlüsseln enthalten die Daten jedoch weitere Informationen, die es entweder der anfragenden Person ermöglichen, den Primärschlüssel des Patienten zweifelsfrei aufzufinden oder es sogar dem Portal ermöglichen, die anfragende Person zu identifizieren und hier den Primärschlüssel zu übermitteln, so dass der Patient letztendlich identifiziert wird. Für die weiteren Informationen bieten sich dabei mehrere vorteilhafte Möglichkeiten an.

In einem nicht erfindungsgemäßen Ausführungsbeispiel kann vorgesehen sein, dass die Daten eine eindeutige Kennung umfassen, welche Kennung von der Kommunikationseinrichtung an das Portal gesendet wird, welches in Antwort den Primärschlüssel an die Kommunikationseinrichtung übermittelt. Bei einer solchen eindeutigen Kennung kann es sich beispielsweise um eine so genannte "Challenge" handeln. Beispielsweise wird eine systemweit eindeutige Zufallszahl generiert, die an das Portal übermittelt wird. Das Portal ermittelt anhand des oder der Sekundärschlüssel den Primärschlüssel, der dem jeweiligen Patienten zugeordnet ist und ordnet dem jeweiligen Patienten, gegebenenfalls nur temporär, um einen Missbrauch zu vermeiden, diese Zufallszahl, im Allgemeinen die eindeutige Kennung, zu. Von der Kommunikationseinrichtung aus kann dann dieselbe Zufallszahl bzw. die Kennung als eine Anfrage an das Portal gesendet werden. Die Kommunikationseinrichtung fragt also an, wer kürzlich die entsprechende Zufallszahl bzw. eindeutige Kennung an das Portal übermittel hat. Das Portal, das die Kennung dem Primärschlüssel zugeordnet abgelegt hat, kann nun den Primärschlüssel an die anfragende Kommunikationseinrichtung senden. Die Übertragung der eindeutigen Kennung von dem Mobilterminal an die Kommunikationseinrichtung kann auf verschiedene denkbare Arten erfolgen, beispielsweise informell, indem der Patient der anfragenden Person diese mitteilt.

Dabei ist es in diesem Fall unerheblich, ob der Patient ansprechbar ist oder nicht. Es kann beispielsweise vorgesehen sein, dass für Notfallsituationen eine vorgeschriebene SMS oder ein Softwaremittel auf dem Mobilterminal vorgesehen ist, welches entsprechende Daten, die die Kennung und den Sekundärschlüssel umfassen, auf einfache Art und Weise erzeugt und an das Portal übermittelt. So kann auch ein nicht ansprechbarer Patient, dessen Mobilterminal bzw. SIM-Karte voll funktionsfähig ist, identifiziert werden.

Als Alternative für die eindeutige Kennung ist es denkbar, GPS-Koordinaten zu verwenden. Dafür müssen sowohl das Mobilterminal als auch die Kommunikationseinrichtung mit einem GPS-Sensor ausgestattet sein und sich am selben Ort befinden. Dann können als Daten der oder die Sekundärschlüssel zusammen mit den GPS-Koordinaten als eindeutige Kennung an das Portal übermittelt werden, wonach sich die Kommunikationseinrichtung ohne weiteren Datenaustausch, da sie sich am selben Ort befindet, über beispielsweise mittels Knopfdruck übersendbare GPS-Koordinaten identifizieren kann und den Primärschlüssel erhält. Dies ist vor allem in Notfallsituationen, in denen ein schneller Zugriff auf die elektronische Patientenakte und daher eine schnelle Identifikation des Patienten gewünscht ist, sehr vorteilhaft, da kein informeller oder anders gearteter Austausch der Kennung zwischen dem Mobilterminal und der Kommunikationseinrichtung stattfinden muss.

Erfindungsgemäß umfassen die Daten Identifikationsdaten der anfragenden Person, anhand welcher das Portal die Kommunikationseinrichtung ermittelt und den Primärschlüssel an die Kommunikationseinrichtung übermittelt. Die anfragende Person besitzt demnach eine für sie personalisierte Kommunikationseinrichtung, und in den Daten sind Identifikationsdaten zur Identifikation der anfragenden Person enthalten. Bei diesen Identifikationsdaten kann es sich beispielsweise um eine Nummer zur Identifizierung eines Arztes bzw. sonstigen Heilberuflers handeln. Jede anfragende Person besitzt dabei eine personalisierte Kommunikationseinrichtung, die ihr zuordenbar ist. Das kann beispielsweise der Arbeitsplatz-PC eines Arztes, aber auch ein Gerät sein, das ein Rettungssanitäter zum Zugriff auf Patientenakten bei Einsätzen mit sich führt.

Besonders vorteilhaft ist es, wenn die anfragende Person selbst in der bzw. in den Datenbanken erfasst ist, ihr mithin auch ein Sekundärschlüssel zugeordnet ist, welche eine einen Teilnehmer eines drahtlosen Kommunikationsnetzes kennzeichnende Teilnehmerinformation ist. Unter diesen Umständen sind, insbesondere für Notfälle, zwei Ausgestaltungen des Verfahrens denkbar, die es ermöglichen, ohne weitere, gegebenenfalls zeitaufwändige Eingaben, die Identifikationsdaten und den oder die auf den Patienten bezogenen Sekundärschlüssel als Daten an das Portal zu übertragen. So ist erfindungsgemäß in einer ersten Alternative vorgesehen, dass die SIM-Karte des Patienten in einem Mobilterminal der anfragenden Person eingesetzt wird und der SIM-kartenbezogene Sekundärschlüssel zur Ermittlung des Primärschlüssels und die Mobilterminalgerätenummer, gegebenenfalls als Sekundärschlüssel, zur Ermittlung der anfragenden Person und somit der Kommunikationseinrichtung dient. Ist demnach das Mobilterminal des Patienten nicht verfügbar bzw. defekt, so kann der Arzt zur Anfrage sein Mobilterminal verwenden. Dabei sind mehrere Ausgestaltungen denkbar. Ist der Patient ansprechbar, so kann er seine Pin zur Aktivierung der SIM-Karte im Mobilterminal der anfragenden Person dieser mitteilen. In einer Alternative kann vorgesehen sein, dass - durch entsprechende Anpassung der Datenstruktur auf der SIM-Karte bzw. durch eine entsprechende Ausbildung des Mobilterminals - neben der Eingabeaufforderung zur Eingabe der Pin zusätzlich eine Notfalloption besteht, deren Aktivierung beispielsweise eine vordefinierte SMS, ein GPRS-Paket oder dergleichen, das die Identifikationsdaten und die Sekundärschlüssel des Patienten umfasst, an das Portal gesendet wird. In einer dritten Ausgestaltung ist es ebenso denkbar, dass das Mobilterminal der anfragenden Person mehrere Steckplätze für SIM-Karten besitzt und die SIM-Karte des Patienten zur Erlangung der oder des Sekundärschlüssels nach Einsetzen in einen der zusätzlichen Steckplätze auslesbar ist.

Alternativ ist erfindungsgemäß vorgesehen, dass die SIM-Karte der anfragenden Person in einem Mobilterminal des Patienten eingesetzt wird und eine SIM-kartenbezogene Information, gegebenenfalls als Sekundärschlüssel, zur Ermittlung der anfragenden Person und somit der Kommunikationseinrichtung und die Mobilterminalgerätenummer als Sekundärschlüssel zur Ermittlung des Primärschlüssels dient. Diese Ausführungsform erfordert im Gegensatz zur vorgenannten keine Kommunikation zwischen Patient und anfragender Person bzw. keine Modifikation des Mobilterminals, sondern kann bei funktionsfähigem Mobilterminal des Patienten unabhängig von dessen Zustand genutzt werden, da die SIM-Karte der anfragenden Person, insbesondere des Heilberuflers, verwendet wird. Die anfragende Person übersendet dann beispielsweise eine vordefinierte SMS, die auch die IMEI-Nummer des Mobilterminals enthält, an das Portal und identifiziert damit sich und den Patienten, so dass er dessen Primärschlüssel an seine Kommunikationseinrichtung erhält.

Wie schon erwähnt, sind die Ausgestaltungen, bei welchen Identifikationsdaten der anfragenden Person mit übersendet werden, insbesondere dann zweckmäßig, wenn die anfragende Person selber durch einen entsprechenden Sekundärschlüssel im Datenbanksystem identifizierbar ist. In den genannten Fällen ist es dann jedoch notwendig, auf jeden Fall mehrere Sekundärschlüssel zu verwenden, beispielsweise die Mobilterminalgerätenummer und die Rufnummer und/oder die SIM-Kartennummer.

Die Daten können jedoch in einer nicht erfindungsgemäßen Ausgestaltung auch als eine Zugriffsanfrage ausgebildet sein. Dabei kann von dem Patienten oder der anfragenden Person von dem Mobilterminal eine den oder die Sekundärschlüssel umfassende Zugriffsanfrage an das Portal gesendet werden, worauf das Portal an das Mobilkommunikationsterminal einen dem über den oder die Sekundärschlüssel ermittelten Primärschlüssel zugeordneten Identifikationsschlüssel übermittelt, welcher Identifikationsschlüssel über die Kommunikationseinrichtung an das Portal gesendet wird, worauf das Portal der Kommunikationseinrichtung den Primärschlüssel übermittelt. Hierbei ist eine etwas sicherere Variante vorgeschlagen, wobei - beispielsweise wieder durch eine entsprechende Option auf dem Mobilterminal - eine SMS an das Portal geschickt wird, welche mindestens die oder den Sekundärschlüssel umfasst. Alternativ sind auch hier selbstverständlich GRPS, WAP oder ähnliche Übertragungsmöglichkeiten denkbar. Statt einer Übermittlung des Primärschlüssels auf unmittelbarem Weg wird nun jedoch durch das Portal dem Primärschlüssel ein Identifikationsschlüssel zugeordnet, welcher an dasselbe Mobilterminal, von dem aus die Anfrage getätigt wurde, zurück übersandt wird. Dieser Identifikationsschlüssel entspricht dann im Prinzip einer Art Identifizierungscode, der nur mit einer entsprechend ausgebildeten Kommunikationseinrichtung nutzbar ist. Er kann somit als eine Art Zugriffscode ausgestaltet werden.

In weiterer nicht erfindungsgemäßer Ausbildung kann ein zeitlich beschränkt gültiger Identifikationsschlüssel verwendet werden. Hierüber ist es beispielsweise möglich, auch einen zeitlich beschränkten Zugang zur elektronischen Patientenakte des Patienten zu regeln. Zudem wird ein Missbrauchsrisiko reduziert. Der ansprechbare Patient kann den Identifikationsschlüssel beispielsweise für einen ihn behandelnden Heilberufler anfordern und diesen an den Heilberufler weitergeben. Nur ein Heilberufler mit einer Kommunikationseinrichtung kann diesen dann nutzen. Dasselbe gilt jedoch, wenn der Patient nicht ansprechbar ist, und beispielsweise ein Heilberufler oder eine andere anfragende Person in einem Notfall einen Identifikationsschlüssel erfragen möchte.

Die bisher genannten Möglichkeiten entsprechen einer Vorwärtsidentifikation. Dabei muss nicht einmal vermutungsweise bekannt sein, um wen es sich bei dem Patienten handelt, das heißt, welcher Primärschlüssel ihm zugeordnet ist. Im Rahmen des erfindungsgemäßen Verfahrens ist jedoch auch eine so genannte Rückwärtsidentifikation möglich. Bei einer Rückwärtsidentifikation wird vermutet, beispielsweise anhand des Namens und des Geburtsdatums des Patienten, wer der Patient sein könnte, das heißt, welcher Primärschlüssel dem Patienten zugeordnet ist. Diese vermutete Identität soll dann zur sicheren Identifikation des Patienten überprüft werden.

Dazu kann in einer nicht erfindungsgemäßen Ausgestaltung vorgesehen sein, dass durch die anfragende Person mittels der Kommunikationseinrichtung einen vermuteten Primärschlüssel des Patienten umfassende Daten an das Portal gesendet werden, worauf das Portal eine Nachricht, insbesondere eine SMS, an eine als Sekundärschlüssel genutzte Rufnummer eines anhand des Primärschlüssels ermittelten Patienten schickt. Alternativ ist auch denkbar, dass das Portal insbesondere automatisiert bei dem Patienten anruft. Der Inhalt einer solchen Nachricht kann dabei beliebig ausgestaltet sein. Die anfragende Person stellt letztendlich durch Nachfrage beim Patienten oder durch einen entsprechenden Blick auf das Mobilterminal des Patienten bzw. das die SIM-Karte des Patienten enthaltende Mobilterminal fest, ob der Primärschlüssel, den er aufgefunden hat, der richtige Primärschlüssel ist. Das Verfahren bietet hierbei somit eine Rückversicherungsmethode, durch die Verwechslungen praktisch ausgeschlossen werden können. Geht keine derartige Nachricht bzw. ein derartiger Anruf bei dem Patienten ein, so ist bekannt, dass der Primärschlüssel offenbar nicht korrekt ist.

Der Sekundärschlüssel muss zum Auffinden des Primärschlüssels jedoch nicht zwangsläufig über das drahtlose Kommunikationsnetzwerk, auf das die Teilnehmerinformation bezogen ist, übertragen werden. In einer weiteren nicht erfindungsgemäßen Ausgestaltung des Verfahrens ist auch denkbar, dass der oder die Sekundärschlüssel über eine drahtlose Kommunikationsverbindung, insbesondere eine Infrarot- oder Bluetoothverbindung, zwischen dem Mobilterminal und der Kommunikationseinrichtung übertragen werden, worauf die Kommunikationseinrichtung den Sekundärschlüssel an das Portal sendet, das Portal aus dem Sekundärschlüssel den Primärschlüssel ermittelt und an die Kommunikationseinrichtung übermittelt. Hierzu ist wiederum eine entsprechende Ausbildung des Mobilterminals erforderlich, die durch das Zur-Verfügung-Stellen eines entsprechenden Softwaremittels realisiert werden kann. Ein Patient löst dann die entsprechende Funktion an seinem Mobilterminal aus, worauf eine entsprechende Verbindung, beispielsweise eine Bluetooth- oder Infrarotverbindung, aufgebaut wird, über die der oder die Sekundärschlüssel übertragen werden. Sind der oder die Sekundärschlüssel der anfragenden Person erst bekannt und auf der Kommunikationseinrichtung vorhanden, so können diese an das Portal gesendet werden, welches den zugehörigen Primärschlüssel ermittelt und an die Kommunikationseinrichtung zurück übermittelt, sodass die anfragende Person die entsprechende Identität des Patienten erfährt und gegebenenfalls Zugriff auf dessen elektronische Patientenakte nehmen kann.

An dieser Stelle sein angemerkt, dass, z. B. zur Realisierung der entsprechenden Datenschutzbestimmungen, zum tatsächlichen Zugriff auf die jeweilige elektronische Patientenakte weitere Sicherheitsvorkehrungen getroffen sein können. Gegebenenfalls ist jedoch schon das Erfahren des Primärschlüssels als kritisch einzustufen. In jedem Fall kann mit Aufnahme des Patienten in das System eine Ermächtigung vorgesehen sein, mittels welcher der Patient Personen oder Personengruppen, gegebenenfalls bei Eintritt besonderer Umstände, zum Zugriff auf Informationen, insbesondere den Primärschlüssel und die elektronische Patientenakte selber, ermächtigt. Im Falle einer Vorwärtsidentifikation bei ansprechbaren Patienten kann eine fallweise Freigabe des Primärschlüssels/der elektronischen Patientenakte mittels der durch das Verfahren ermöglichten Kombination der Identität der anfragenden Person, insbesondere des Heilberuflers, vorzugsweise ebenso über den oder die Sekundärschlüssel, mit einer dafür vorgesehenen PIN, Signatur oder einem ähnlichen Code des Patienten erreicht werden. Für beispielsweise Notfälle ist es jedoch insbesondere zweckmäßig, wenn eine zuvor abgelegte generelle Freigabe der elektronischen Patientenakte bzw. des Primärschlüssels in Form einer Ermächtigung vorliegt, die jedoch nur für bestimmte anfragende Personen, insbesondere Heilberufler, die in Notfällen schnell handeln müssen, vorgesehen ist. Durch die Identität des Heilberuflers bzw. die Nutzung der entsprechenden Kommunikationseinrichtung kann diesem dann der entsprechende Zugang gewährt werden.

Die bislang diskutierten Fälle beschäftigen sich im Wesentlichen mit einem ansprechbaren Patienten bzw. dessen funktionstüchtigem Mobilterminal oder einer SIM-Karte, die auch nach Einsetzen in ein Fremdmobilterminal geeignet ist, eine entsprechende Notfallübersendung einzuleiten. Es ist jedoch auch denkbar, dass auf der SIM-Karte ein Notschlüssel abgelegt ist. Dieser auf der SIM-Karte des Patienten abgelegte Notschlüssel kann von der Kommunikationseinrichtung der anfragenden Person dann ausgelesen und an das Portal übertragen werden, worauf das Portal anhand des als Sekundärschlüssel dienenden Notschlüssels den Primärschlüssel des Patienten ermitteln kann und an die Kommunikationseinrichtung übermitteln kann. Dann kann in jedem Falle der Primärschlüssel ermittelt und somit der Patienten identifiziert werden.

Ist im Übrigen auch die SIM-Karte des Patienten defekt, so findet sich die SIM-Kartennummer meist auch auf der SIM-Karte selber abgedruckt, sodass dieser Sekundärschlüssel trotzdem ermittelt werden kann. Ähnliches gilt gegebenenfalls auch für die IMEI-Nummer.

Da auch Mobilterminals und SIM-Karten, teilweise unfreiwillig, ihren Besitzer wechseln können, ohne dass dies angezeigt wird, ist es besonders vorteilhaft, wenn eine weitere Überprüfung der Identität eines Patienten stattfinden kann. Dazu kann erfindungsgemäß beispielsweise vorgesehen sein, dass eine weitere Identitätsverifikation anhand biometrischer Merkmale und/oder eines Bildes des Patienten, die und/oder das zwischen dem Portal und dem Mobilterminal und/oder der Kommunikationseinrichtung ausgetauscht werden, erfolgt. Dabei wird dementsprechend überprüft, ob der eigentliche Patient zu dem bei ihm gefundenen Mobilterminal bzw. der bei ihm gefundenen SIM-Karte gehört.
Konkret kann hierbei vorgesehen sein, dass das Mobilterminal und/oder die Kommunikationseinrichtung mit biometrischer Sensorik und/oder einer Kamera ausgestattet ist. Dann kann ein Bild oder biometrische Daten des Patienten an das Portal übersendet werden und mit dort, insbesondere in der zweiten Datenbank, abgelegten biometrischen Daten oder einem dort abgelegten Bild verglichen werden und das Ergebnis des Vergleichs an das Mobilterminal und/oder die Kommunikationseinrichtung übermittelt werden. Die durch den oder die Sekundärschlüssel ermittelte Identität des Patienten wird also zusätzlich entweder durch biometrische Merkmale, beispielsweise einen Fingerabdruck oder einen Retinascan, oder auch ein Bild überprüft, die jeweils datenbankseitig mit zuvor abgelegten biometrischen Daten bzw. einem Bild verglichen werden. Das Ergebnis dieses Vergleiches wird an die Einrichtung zurück übersandt, welche die zur Überprüfung verwendeten biometrischen Daten bzw. das zur Überprüfung verwendete Bild eingesendet hat. Damit wird verhindert, dass beispielsweise bei einem ausgeliehenen Handy falsche Gesundheitsdaten aus einer elektronischen Patientenakte, die gar nicht zu dem aufgefundenen Patienten gehört, mit gegebenenfalls sogar kritischen Folgen verwendet wird.

Alternativ zu einer Ausstattung des Mobilterminals oder der Kommunikationseinrichtung mit einer Kamera oder entsprechenden biometrischen Sensoren kann auch vorgesehen sein, dass mit oder vor Übermittlung eines Primärschlüssels ein zuvor in der bzw. der zweiten Datenbank abgelegtes Bild des zugehörigen Patienten an die Kommunikationseinrichtung gesendet wird. Die anfragende Person erhält dabei auch ein Bild des Patienten, das sie mit dem Aussehen der zu identifizierenden Person vergleichen kann. Auf diese Weise ist eine besonders einfache Identitätsverifikation ermöglicht, die Fehlzuordnungen von Primärschlüsseln zu Patienten weiter reduziert. Insbesondere kann vorgesehen sein, dass zunächst nur das Bild des Patienten an die Kommunikationseinrichtung gesendet wird. In diesem Fall wird der Primärschlüssel erst nach einem bestätigten Vergleich des Bildes mit einer der Patient zu sein scheinenden Person übermittelt. Dazu wird dann eine entsprechende Bestätigung von der Kommunikationseinrichtung an das Portal übersendet, welche dann den Primärschlüssel übersendet. Hier ist die Identitätsverifikation als eindeutige Stufe in dem Identifikationsprozess vorgesehen, die erst bewältigt werden muss, bevor der Primärschlüssel der anfragenden Person zur Verfügung gestellt wird.

Das erfindungsgemäße Verfahren bietet also unter Nutzung schon vorhandener Infrastrukturen und einer ohnehin vorhandenen Personalisierungsstufe, nämlich der Teilnehmerinformation, eine Möglichkeit zur Identifikation eines Patienten, also zum Auffinden eines Primärschlüssels, bei dem weitere Einrichtungen oder Personalisierungsstufen nicht mehr notwendig sind. Insbesondere kann der Grundgedanke der Erfindung in diese Richtung vorteilhaft erweitert werden. So kann beispielsweise die SIM-Karte des Patienten eine bislang vorhandene Krankenversicherungskarte auch ersetzen, da die SIM-Karte für den entsprechenden Patienten auch personalisiert ist und diesen eindeutig identifiziert. Durch die Verknüpfung von auf die SIM-Karte bezogenen Sekundärschlüsseln mit dem Primärschlüssel der elektronischen Patientenakte ist es dann auch ohne eine elektronische Krankenversicherungskarte möglich, den Patienten einer Patientenakte und somit auch einer Krankenkasse zuzuordnen. Zudem sind die Speichermöglichkeiten auf einer SIM-Karte auch für solche Informationen nutzbar.

In einer anderen Weiterbildung des Erfindungsgedankens ist es auch denkbar, dass eine Abrechnung medizinischer Dienste über einen Mobilkommunikationsprovider erfolgen kann. Da jeder Patient im drahtlosen Kommunikationsnetz des Mobilkommunikationsproviders identifizierbar ist und auch entsprechend über das Portal, das ja beispielsweise im Einflussbereich des Mobilkommunikationsproviders angeordnet sein kann, gehandhabt wird, ist es auch denkbar, die medizinischen Dienste hierüber abzurechnen, wodurch ein gewisser Verwaltungsaufwand entfällt.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: Komponenten eines Systems, in dem das erfindungsgemäße Verfahren angewendet wird,
- Fig. 2: einen ersten Ablaufplan des erfindungsgemäßen Verfahrens,
- Fig. 3: einen zweiten Ablaufplan eines nicht erfindungsgemäßen Verfahrens, und
- Fig. 4: einen Ablaufplan der Identitätsverifikation gemäß des erfindungsgemäßen Verfahrens.

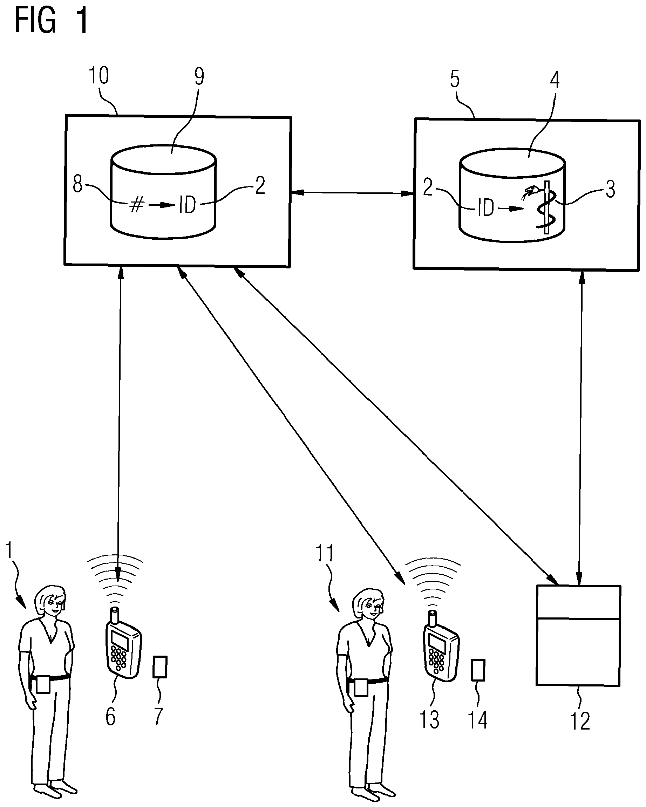

Das erfindungsgemäße Verfahren soll nun anhand einiger Ausführungsbeispiele näher erläutert werden. Zunächst zeigt Fig. 1 wichtige Komponenten eines Systems, in dem das erfindungsgemäße Verfahren ausgeführt wird. Dabei deuten die Pfeile zwischen den Komponenten mögliche Kommunikationswege an; es sei jedoch angemerkt, dass nicht jeder der angedeuteten Kommunikationswege in jeder der Ausführungsformen in jeder Richtung Verwendung findet.

Identifiziert werden soll durch das erfindungsgemäße Verfahren ein Patient 1, das bedeutet, es soll der Primärschlüssel 2 gefunden werden, der eine elektronische Patientenakte 3 mit Gesundheitsdaten des Patienten in einer ersten Datenbank 4, die beispielsweise auf einen Server 5 abgelegt ist, kennzeichnet. Der Patient 1 ist ein Teilnehmer eines drahtlosen Kommunikationsnetzwerks. Zur Kommunikation in diesem besitzt er ein Mobilterminal 6, hier ein Mobiltelefon, das eine Mobilterminalgerätenummer, die eindeutig ist, insbesondere eine IMEI, aufweist. Zudem besitzt der Patient 1 eine SIM-Karte 7, die eine ebenfalls eindeutige SIM-Kartennummer aufweist und dem Patienten 1 eine eindeutige Rufnummer zuweist. Durch die Mobilterminalgerätenummer, die Rufnummer und die SIM-Kartennummer ist eine Personalisierungsstufe des Patienten 1 gegeben, durch die der Patient 1 eindeutig identifizierbar wird. Deshalb werden eine oder mehrere dieser Teilnehmerinformationen als ein Sekundärschlüssel 8 verwendet, der in einer zweiten Datenbank 9 dem entsprechenden Primärschlüssel 2 eindeutig zugeordnet ist. Die zweite Datenbank 9 ist in einem Portal 10 abgelegt. Es ist jedoch auch möglich, dass die erste Datenbank 4 und die zweite Datenbank 9 als eine einzige Datenbank realisiert ist, die dann auch im Portal bzw. einem mit dem Portal kommunizierenden Server abgelegt sein kann. Im vorliegenden Fall ist das Portal 10 der Einflusssphäre eines Mobilkommunikationsproviders zugeordnet, während die elektronischen Patientenakten auf einem weiteren, hierzu externen Server 5 verwaltet werden.

Eine anfragende Person 11, insbesondere ein Heilberufler, möchte den Patienten 1 eindeutig identifizieren, das bedeutet, seinen Primärschlüssel 2 erfahren, um später mittels einer dazu ausgebildeten Kommunikationseinrichtung 12 auf die elektronische Patientenakte 3 zugreifen zu können. Auch die anfragende Person 11 kann ein Mobilterminal 13 und eine SIM-Karte 14 besitzen, die eine entsprechende Personalisierungsstufe für sie darstellen.

Dabei ist anzumerken, dass auch die Kommunikationseinrichtung 12 selbst ein Mobilterminal sein kann, das heißt, die Kommunikationseinrichtung 12 und das Mobilterminal 13 müssen nicht getrennte Einheiten darstellen.

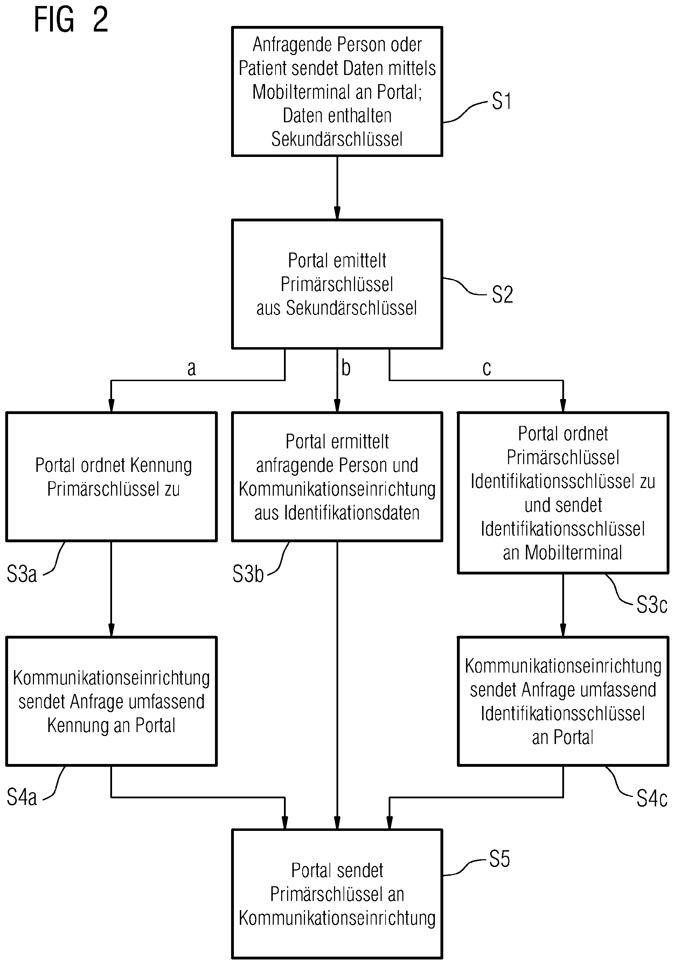

Fig. 2 zeigt nun einen ersten Ablaufplan, in dem Ausführungsformen des Verfahrens zur Vorwärtsidentifikation näher erläutert werden.

Zunächst werden in Schritt S1 Daten mittels des Mobilterminals 6 oder 13 an das Portal 10 gesendet. Diese Daten enthalten wenigstens einen Sekundärschlüssel 8 des Patienten 1. Wer das Senden der Daten auslöst, hängt im Wesentlichen davon ab, ob der Patient 1 ansprechbar ist. Das Senden der Daten kann sowohl vom Patienten 1 als auch von der anfragenden Person 11 initiiert werden.

Die Daten können auf verschiedene Weise gesendet werden, beispielsweise in Form einer SMS, mittels GPRS oder WAP oder anderer Übermittlungstechniken. Relevant ist, dass wenigstens ein Sekundärschlüssel 8 des Patienten 1 darin enthalten ist. Eine Rufnummer wird bei Verwendung der SIM-Karte 7 des Patienten 1 ohnehin übersendet, andere Sekundärschlüssel können beispielsweise mittels eines geeigneten Softwaremittels ermittelt und in die Daten eingefügt werden. Es kann auch eine vorformulierte SMS vorgesehen sein. Insgesamt sind viele Ausgestaltungsmöglichkeiten denkbar.

Die Daten werden von einem Mobilterminal, also dem Mobilterminal 6 des Patienten 1, dem Mobilterminal 13 der anfragenden Person 11 oder einem weiteren Mobilterminal gesendet. Da wenigstens ein Sekundärschlüssel 8 des Patienten 1 benötigt wird, sind folgende Konstellationen denkbar und sinnvoll. Zum einen kann natürlich das Mobilterminal 6 des Patienten mit der SIM-Karte 7 des Patienten verwendet werden. Es kann jedoch auch das Mobilterminal 6 des Patienten mit der SIM-Karte 14 der anfragenden Person 11 oder einer anderen SIM-Karte verwendet werden. Genauso kann das Mobilterminal 13 der anfragenden Person 11 oder ein anderes Mobilterminal mit der SIM-Karte 7 des Patienten 1 verwendet werden. In allen diesen Fällen steht wenigstens einer der Sekundärschlüssel zur Verfügung.

Die Daten werden in Schritt S2 von dem Portal 10 empfangen, welches den Primärschlüssel 2 aus dem Sekundärschlüssel 8, der ja in den Daten enthalten ist, in der Datenbank 9 ermittelt.

Im weiteren Verlauf des Verfahrens sind mehrere Ausgestaltungen einer Vorwärtsidentifikation denkbar, die in Fig. 2 mit a, b und c gekennzeichnet sind.

Im nicht erfindungsgemäßen Fall a umfassen die an das Portal 10 übersandten Daten zusätzlich eine eindeutige Kennung in Bezug auf das Portal 10. Dies kann beispielsweise im Rahmen einer so genannten "Challenge" eine eindeutige Zufallszahl sein. Es ist jedoch auch denkbar, dass GPS-Koordinaten des entsprechenden Mobilterminals 6 oder 13, gegebenenfalls mit einem Zeitrahmen oder Zeitstempel, verwendet werden. Nach der Ermittlung des Primärschlüssels 2 aus dem Sekundärschlüssel 8 ordnet das Portal 10 in Schritt S3a die ebenso in den Daten enthaltene Kennung dem Primärschlüssel 2 zu. Von der Kommunikationseinrichtung 12 aus kann dann durch die anfragende Person 11 eine Anfrage, die die Kennung umfasst, an das Portal 10 gesendet werden. Dies geschieht im Schritt S4a. Aufgrund der Kennung kann das Portal 10 den Primärschlüssel 2 ermitteln und in Schritt S5 der Kommunikationseinrichtung 12 übersenden. Selbstverständlich ist dabei dem Portal 10 auch bekannt, woher die Anfrage kam, sodass hierdurch die Kommunikationseinrichtung 12 identifiziert wird und der Primärschlüssel 2 an sie übersandt werden kann.

In der erfindungsgemäßen Ausführungsform b umfassen die an das Portal 10 übersendeten Daten Identifikationsdaten, die zur Identifikation der anfragenden Person 11 und somit der ihr persönlich zugeordneten Kommunikationseinrichtung 12 dienen. Selbstverständlich ist es auch möglich, Identifikationsdaten mit zu übersenden, die unmittelbar der Identifikation der Kommunikationseinrichtung 12 dienen. Anhand dieser Identifikationsdaten ermittelt das Portal 10 in Schritt S3b die anfragende Person 11 und/oder die Kommunikationseinrichtung 12 anhand der Identifikationsdaten. Besonders vorteilhaft ist es hierbei, wenn auch die anfragende Person 12 im Datenbanksystem vorhanden ist, sodass auch hier Sekundärschlüssel zugeordnet sind, die die anfragende Person 11 als Teilnehmer des drahtlosen Kommunikationsnetzes kennzeichnen. Insbesondere dann sind zwei einfache, alternative Arten denkbar, wie eine gleichzeitige Identifizierung des Patienten 1 und der anfragenden Person 11 und somit der Kommunikationseinrichtung 12 erfolgen kann. Zum einen ist es möglich, dass die SIM-Karte 14 der anfragenden Person 11 in das Mobilterminal 6 des Patienten 1 eingesteckt ist. Anhand des oder der Sekundärschlüssel, SIM-Kartennummer und/oder Rufnummer, kann die anfragende Person 11 identifiziert werden, anhand der Mobilterminalgerätenummer des Mobilterminals 6 als Sekundärschlüssel kann der Patient 1 ermittelt werden. Es ist natürlich auch möglich, dass die SIM-Karte 7 des Patienten 1 in das Mobilterminal 13 der anfragenden Person 11 eingesetzt wird. Dann kann die anfragende Person 11 über die Mobilterminalgerätenummer des Mobilterminals 13 ermittelt werden, der Patient 1 über die SIM-Kartennummer der SIM-Karte 7 und/oder der Rufnummer. Selbstverständlich ist auch denkbar, dass als Identifikationsdaten eine spezielle Heilberuflerkennung übersendet wird, beispielsweise eine Codenummer, mit der sich die anfragende Person 11 gegenüber dem Portal 10 zu erkennen gibt.

Ist die anfragende Person 11 und damit die Kommunikationseinrichtung 12 identifiziert, so übermittelt das Portal 10 den Primärschlüssel 2 des Patienten 1 in Schritt S5 wiederum an die Kommunikationseinrichtung 12.

In einer weiteren nicht erfindungsgemäßen Variante, Variante c, wird lediglich der Sekundärschlüssel 8 in den Daten übertragen. Das Portal 10 ordnet dann in Schritt S3c dem Primärschlüssel 2 einen eindeutigen, generierten Identifikationsschlüssel zu, welcher Identifikationsschlüssel an das jeweilige Mobilterminal 6 oder 13 zurück übertragen wird. Ist der Patient 1 der Anfragende, so kann der Identifikationsschlüssel im Rahmen eines informellen Austausches zu der anfragenden Person 11 und damit der Kommunikationseinrichtung 12 übertragen werden. Ist der Nutzer des anfragenden Mobilterminals 6 oder 13 die anfragende Person 11 selbst, so erlangt diese unmittelbar Kenntnis von dem Identifikationsschlüssel. Dieser Identifikationsschlüssel kann zur Vermeidung von Missbrauch beispielsweise zeitlich begrenzt gültig sein.

Die Kommunikationseinrichtung 12 sendet dann im Schritt S4c eine Anfrage umfassend den Identifikationsschlüssel an das Portal 10. Das Portal 10 kann mittels des Identifikationsschlüssels den Primärschlüssel 2 des Patienten 1 wiederum ermitteln und übersendet diesen in Schritt S5 wiederum an die Kommunikationseinrichtung 12.

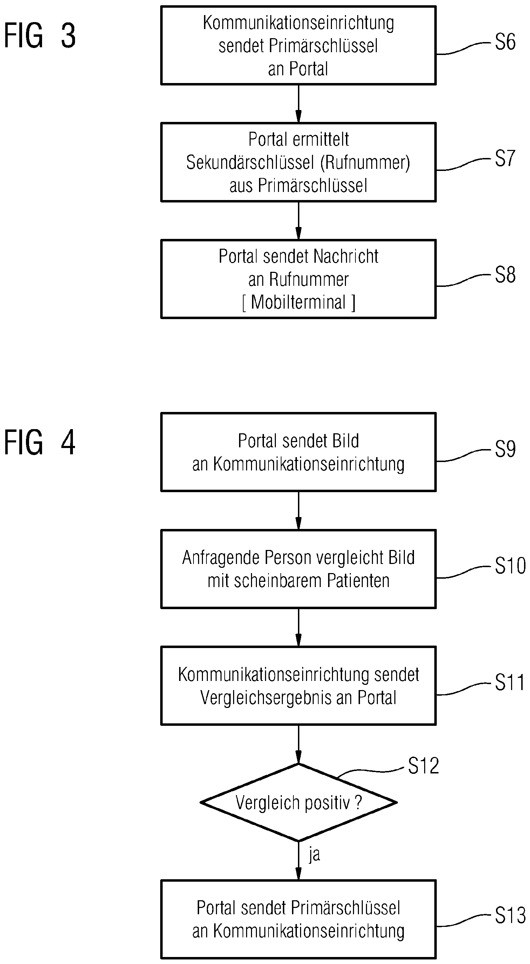

Mittels einer nicht erfindungsgemäßen Ausgestaltung des Verfahrens ist jedoch auch eine Rückwärtsidentifikation möglich. Dies ist in Fig. 3 in einem zweiten Ablaufplan näher dargestellt. Eine Rückwärtsidentifikation bedeutet, dass die anfragende Person 11 bereits einen Primärschlüssel 2 für den Patienten 1 vermutet und diesen Primärschlüssel 2 bestätigt haben möchte. Dazu wird in Schritt S6 von der Kommunikationseinrichtung 12 der Primärschlüssel innerhalb einer Anfrage an das Portal 10 geschickt. In Schritt S7 ermittelt das Portal 10 Sekundärschlüssel 8, insbesondere die Rufnummer, aus dem Primärschlüssel 2. Da nun die Rufnummer des Patienten 1 bekannt ist, kann das Portal 10 in Schritt S8 eine Nachricht an diese Rufnummer senden, die dann jenes Mobilterminal erreicht, in das die SIM-Karte 7 des Patienten 1 eingesteckt ist, im üblichen Fall also das Mobilterminal 6. Der Patient 1 bzw. die anfragende Person 11 können nun ob des Eingangs der Nachricht überprüfen, ob der Primärschlüssel 2 tatsächlich dem Patienten 1 zugeordnet ist.

Fig. 4 zeigt schließlich eine Möglichkeit zu einer weiteren Identitätsverifikation im Rahmen des erfindungsgemäßen Verfahrens. Die in Fig. 4 dargestellten Schritte S9 bis S13 werden beispielsweise vor Schritt S5 in Fig. 2 durchgeführt. Dabei sendet das Portal 10 nicht unmittelbar den Primärschlüssel 2 an die Kommunikationseinrichtung 12, sondern sendet zunächst ein zuvor im Portal 10, insbesondere in der zweiten Datenbank 9, abgelegtes Bild des Patienten 1 an die Kommunikationseinrichtung 12. Dies geschieht in Schritt S9. Die anfragende Person 11 kann danach das Bild mit dem scheinbaren Patienten 1 vergleichen, um festzustellen, ob es sich bei der Person tatsächlich um den Patienten 1 handelt. Nachdem dies in Schritt S10 geschehen ist, sendet in Schritt S11 die Kommunikationseinrichtung 12 das Vergleichsergebnis an das Portal 10 zurück, worauf in Schritt S12 im Portal 10 überprüft wird, ob der Vergleich positiv oder negativ verlaufen ist. Ist der Vergleich negativ verlaufen, so ist der Primärschlüssel 2 nicht der Person zugeordnet, die von der anfragenden Person 11 für den Patienten 1 gehalten wurde. Es sind dann keine weiteren Maßnahmen erforderlich. Ist die Identität des Patienten 1 jedoch bestätigt, so sendet das Portal in Schritt S13 den Primärschlüssel 2 an die Kommunikationseinrichtung 12.

## Patentansprüche

1. Verfahren zur Identifikation eines Patienten zum späteren Zugriff auf eine elektronische Patientenakte des Patienten mittels einer Kommunikationseinrichtung einer anfragenden Person, welche Patientenakte in einer Datenbank über einen der Identifikation des Patienten dienenden Primärschlüssel, dem eindeutig wenigstens ein Sekundärschlüssel zugeordnet ist, gespeichert ist, wobei die anfragende Person ein Heilberufler ist, wobei als Sekundärschlüssel zur Identifikation eines Patienten wenigstens eine einen Teilnehmer eines drahtlosen Kommunikationsnetzes kennzeichnende Teilnehmerinformation verwendet wird, welcher Sekundärschlüssel zur Identifikation durch Auffinden des Primärschlüssels zwischen einem zur Kommunikation in dem drahtlosen Kommunikationsnetz dienenden Mobilterminal und einem Portal über das oder wenigstens ein Kommunikationsnetz übertragen wird, wobei eine auf dem Portal abgelegte zweite Datenbank verwendet wird, worin der oder die Sekundärschlüssel dem Primärschlüssel zugeordnet sind, und der dem Sekundärschlüssel zugeordnete Primärschlüssel an die Kommunikationseinrichtung übertragen wird, wobei zur Identifikation des Patienten ein Mobilterminal des Patienten oder ein mit einer SIM-Karte des Patienten versehenes Mobilterminal verwendet wird, welches Daten an das Portal sendet und/oder von dem Portal empfängt, wobei von dem Mobilterminal den oder die Sekundärschlüssel umfassende Daten an das Portal übersendet werden, aufgrund des oder der Sekundärschlüssel durch das Portal der Primärschlüssel ermittelt und unter Nutzung weiterer in den Daten umfasster Informationen der Kommunikationseinrichtung übermittelt wird, wobei die Daten Identifikationsdaten der anfragenden Person umfassen, anhand welcher das Portal die Kommunikationseinrichtung ermittelt und den Primärschlüssel an die Kommunikationseinrichtung übermittelt, wobei die SIM-Karte des Patienten in einem Mobilterminal der anfragenden Person eingesetzt wird und der SIM-kartenbezogene Sekundärschlüssel zur Ermittlung des Primärschlüssels und die Mobilterminalgerätenummer zur Ermittlung der anfragenden Person und somit der Kommunikationseinrichtung dient, oder wobei die SIM-Karte der anfragenden Person in einem Mobilterminal des Patienten eingesetzt wird und eine SIM-kartenbezogene Information zur Ermittlung der anfragenden Person und somit der Kommunikationseinrichtung und die Mobilterminalgerätenummer als Sekundärschlüssel zur Ermittlung des Primärschlüssels dient.

2. Verfahren nach Anspruch 1, **dadurch gekennzeichnet, dass** als Sekundärschlüssel eine Nummer einer SIM-Karte und/oder eine Rufnummer des Patienten und/oder eine Gerätenummer eines zur Kommunikation in dem drahtlosen Kommunikationsnetz dienenden Mobilterminals des Patienten verwendet werden.

3. Verfahren nach Anspruch 1 oder 2, **dadurch gekennzeichnet, dass** von dem Mobilterminal den oder die Sekundärschlüssel umfassende Daten in Form einer SMS oder mittels GPRS an das Portal übersendet werden.

4. Verfahren nach einem der vorangehenden Ansprüche, **dadurch gekennzeichnet, dass** die Mobilterminalgerätenummer als Sekundärschlüssel zur Ermittlung der anfragenden Person und somit der Kommunikationseinrichtung dient.

5. Verfahren nach einem der vorangehenden Ansprüche, **dadurch gekennzeichnet, dass** die SIM-kartenbezogene Information als Sekundärschlüssel zur Ermittlung der anfragenden Person und somit der Kommunikationseinrichtung und die Mobilterminalgerätenummer als Sekundärschlüssel zur Ermittlung des Primärschlüssels dient.

6. Verfahren nach einem der vorangehenden Ansprüche, **dadurch gekennzeichnet , dass** eine weitere Identitätsverifikation anhand biometrischer Merkmale und/oder eines Bildes des Patienten, die zwischen dem Portal und dem Mobilterminal und/oder der Kommunikationseinrichtung ausgetauscht werden, erfolgt.

7. Verfahren nach Anspruch 6, **dadurch gekennzeichnet, dass** ein Mobilterminal und/oder eine Kommunikationseinrichtung mit biometrischer Sensorik und/oder einer Kamera verwendet werden.

8. Verfahren nach Anspruch 7, **dadurch gekennzeichnet**, **dadurch gekennzeichnet, dass** ein Bild oder biometrische Daten an das Portal übersendet und mit dort, insbesondere in der zweiten Datenbank, abgelegten biometrischen Daten oder einem dort abgelegten Bild verglichen werden und das Ergebnis des Vergleichs an das Mobilterminal und/oder die Kommunikationseinrichtung übermittelt wird.

9. Verfahren nach Anspruch 6, **dadurch gekennzeichnet, dass** mit oder vor Übermittlung eines Primärschlüssels ein Bild des zugehörigen Patienten an die Kommunikationseinrichtung gesendet wird.

10. Verfahren nach Anspruch 9, **dadurch gekennzeichnet, dass** der Primärschlüssel nach einem bestätigten Vergleich des Bildes mit einer der Patient zu sein scheinenden Person übermittelt wird.

## Claims

1. Method for identifying a patient for later access to an electronic patient record for the patient using a communication device belonging to an inquiring person, which patient record is stored in a database using a primary key which is used to identify the patient and which has at least one explicitly associated secondary key, wherein the inquiring person is a healthcare professional, wherein the secondary key used to identify a patient is at least one subscriber information item which characterizes a subscriber in a wireless communication network, which secondary key for identification by locating the primary key is transmitted between a mobile terminal used for communication in the wireless communication network and a portal via the or via at least one communication network, wherein a second database stored on the portal is used in which the secondary key(s) are associated with the primary key, and the primary key associated with the secondary key is transmitted to the communication device, wherein the patient is identified using a mobile terminal belonging to the patient or a mobile terminal which is provided with a SIM card belonging to the patient and which sends data to the portal and/or receives data from the portal, wherein the mobile terminal sends data comprising the secondary key(s) to the portal, the portal takes the secondary key(s) as a basis for ascertaining the primary key, and further information which the data comprise is used to transmit said primary key to the communication device, wherein the data comprise identification data from the inquiring person which the portal uses to ascertain the communication device and to transmit the primary key to the communication device, wherein the patient's SIM card is used in a mobile terminal belonging to the inquiring person, and the SIM-card-related secondary key is used to ascertain the primary key, and the mobile terminal device number is used to ascertain the inquiring person and hence the communication device, or wherein the inquiring person's SIM card is used in a mobile terminal belonging to the patient, and a SIM-card-related information item is used to ascertain the inquiring person and hence the communication device, and the mobile terminal device number is used as secondary key to ascertain the primary key.

2. Method according to Claim 1, **characterized in that** the secondary key used is a number from a SIM card and/or a telephone number for the patient and/or a device number for a mobile terminal belonging to the patient which is used for communication in the wireless communication network.

3. Method according to Claim 1 or 2, **characterized in that** the mobile terminal sends data comprising the secondary key(s) to the portal in the form of an SMS or by means of GPRS.

4. Method according to one of the preceding claims, **characterized in that** the mobile terminal device number is used as secondary key for ascertaining the enquiring person and hence the communication device.

5. Method according to one of the preceding claims, **characterized in that** the SIM card-related information is used as secondary key for ascertaining the enquiring person and hence the communication device, and the mobile terminal device number is used as secondary key for ascertaining the primary key.

6. Method according to one of the preceding claims, **characterized in that** further identity verification takes place on the basis of biometric features and/or a picture of the patient, these being interchanged between the portal and the mobile terminal and/or the communication device.

7. Method according to Claim 6, **characterized in that** a mobile terminal and/or a communication device having a biometric sensor system and/or a camera are used.

8. Method according to Claim 7, **characterized in that** a picture or biometric data are sent to the portal and compared with biometric data stored there, particularly in the second database, or with a picture stored there, and the result of the comparison is transmitted to the mobile terminal and/or to the communication device.

9. Method according to Claim 6, **characterized in that** a picture of the associated patient is sent to the communication device upon or before transmission of a primary key.

10. Method according to Claim 9, **characterized in that** the primary key is transmitted following confirmed comparison of the picture with a person who appears to be the patient.

## Revendications

1. Procédé d'identification d'un patient pour accéder ultérieurement à un dossier électronique du patient au moyen d'un dispositif de communication d'une personne demandeuse, lequel dossier du patient est mis en mémoire dans une base de données par l'intermédiaire d'une clé primaire, qui sert à l'identification du patient et à laquelle est associée de manière univoque au moins une clé secondaire, la personne demandeuse étant un professionnel de santé, dans lequel on utilise comme clé secondaire pour l'identification d'un patient au moins une information de participant, caractérisant un participant d'un réseau de communication sans fil, laquelle clé secondaire est, pour l'identification en trouvant la clé primaire, transmise par le ou au moins un réseau de communication entre un terminal mobile servant à la communication dans le réseau de communication sans fil et un portail, dans lequel on utilise une deuxième base de données mise en mémoire sur le portail, dans laquelle la ou les clés secondaires sont associées à la clé primaire, et on transmet la clé primaire associée à la clé secondaire au dispositif de communication, dans lequel, pour l'identification du patient, on utilise un terminal mobile du patient ou un terminal mobile pourvu d'une carte SIM du patient, qui envoie des données au portail et/ou en reçoit du portail, dans lequel on transmet du terminal mobile au portail des données comprenant la ou les clés secondaires, on détermine la clé primaire par le portail sur la base de la ou des clés secondaires et, en utilisant d'autres informations comprises dans les données, on la transmet au dispositif de communication, les données comprenant des données d'identification de la personne demandeuse, à l'aide desquelles le portail détermine le dispositif de communication et transmet la clé primaire au dispositif de communication, la carte SIM du patient étant utilisée dans un terminal mobile de la personne demandeuse et la clé secondaire, se rapportant à la carte SIM, servant à la détermination de la clé primaire et le numéro d'appareil du terminal mobile à la détermination de la personne demandeuse et ainsi au dispositif de communication, ou dans lequel on utilise la carte SIM de la personne demandeuse dans un terminal mobile du patient, et l'information, se rapportant à la carte SIM, sert à la détermination de la personne demandeuse et ainsi du dispositif de communication, et le numéro d'appareil du terminal mobile de clé secondaire pour la détermination de la clé primaire.

2. Procédé suivant la revendication 1, **caractérisé en ce que** l'on utilise comme clé secondaire un numéro d'une carte SIM et/ou un numéro d'appel du patient et/ou un numéro d'appareil d'un terminal mobile du patient servant à la communication dans le réseau de communication sans fil.

3. Procédé suivant la revendication 1 ou 2, **caractérisé en ce que** l'on transmet du terminal mobile au portail, sous la forme d'un SMS ou au moyen d'un GPRS, des données comprenant la ou les clés secondaires.

4. Procédé suivant l'une des revendications précédentes, **caractérisé en ce que** le numéro de l'appareil du terminal mobile sert de clé secondaire pour la détermination de la personne demandeuse et ainsi du dispositif de communication.

5. Procédé suivant l'une des revendications précédentes, **caractérisé en ce que** l'information, se rapportant à la carte SIM, sert de clé secondaire pour la détermination de la personne demandeuse et ainsi du dispositif de communication, et le numéro d'appareil du terminal mobile de clé secondaire pour la détermination de la clé primaire.

6. Procédé suivant l'une des revendications précédentes, **caractérisé en ce qu'**une autre vérification d'identité a lieu à l'aide de caractéristiques biométriques et/ou d'une image du patient, qui sont échangées entre le portail et le terminal mobile et/ou le dispositif de communication.

7. Procédé suivant la revendication 6, **caractérisé en ce que** l'on utilise un terminal mobile et/ou un dispositif de communication à capteur biométrique et/ou à caméra.

8. Procédé suivant la revendication 7, **caractérisé en ce que** l'on transmet une image ou des données biométriques au portail et on les compare à des données biométriques, qui sont mises en mémoire, notamment dans la deuxième base de données, ou à une image, qui y est mise en mémoire, et on transmet le résultat de la comparaison au terminal mobile et/ou au dispositif de communication.

9. Procédé suivant la revendication 6, **caractérisé en ce que** l'on envoie au dispositif de communication une image du patient en question avec ou avant la transmission d'une clé primaire.

10. Procédé suivant la revendication 9, **caractérisé en ce que** l'on transmet la clé primaire, après une comparaison confirmée de l'image à l'un des patients, à celle qui semble être la bonne personne.
